# EUROPEAN PATENT SPECIFICATION

(11) **EP 1 863 429 B1**
(45) Date of publication and mention of the grant of the patent: **04.06.2008**
(21) Application number: 06723395.7
(22) Date of filing: 13.03.2006
(51) Int. Cl.: A61K 8/73, A61K 8/64, A61Q 19/00, A61Q 19/10

(54) **COSMETIC DELIVERY SYSTEM AND PROCESS FOR MANUFACTURE THEREOF**
KOSMETISCHES ABGABESYSTEM UND VERFAHREN ZU SEINER HERSTELLUNG
SYSTEME D'APPLICATION DE COSMETIQUES ET PROCEDE DE FABRICATION ASSOCIE

(30) Priority: 30.03.2005 IN MU03682005; 30.03.2005 IN MU03692005; 07.10.2005 EP 05256274
(43) Date of publication of application: 12.12.2007
(73) Proprietor: UNILEVER N.V., 3013 AL Rotterdam (NL); Unilever PLC, Blackfriars London Greater London EC4P 4BQ (GB)
(72) Inventor: BAVISKAR, Rajesh J., Hindustan Lever Research Ctr., Mumbai, Maharashtra 400 099 (IN); SURIANARAYANAN, Ramesh, Hindustan Lever Resch. Ctr, Mumbai,Maharashtra 400 099 (IN); PRADHAN, Ram, Ramesh, Maharashtra, Mumbai 400 075 (IN); KINI, Mridula, Hindustan Lever Research Centre, Mumbai, Maharashtra 400 099 (IN)
(74) Representative: Acham, Nicholas Clive
(86) International application number: PCT/EP2006/002301
(87) International publication number: WO 2006/102985

(56) References cited:
- EP-A- 1 402 790
- US-A- 5 545 414
- US-A1- 2003 004 114
- US-B1- 6 197 319

## Description

### Technical Field of the Invention

The invention relates to a benefit agent delivery system for use in cosmetic or cleansing products. The invention more particularly relates to a delivery system in the form of shear sensitive globules for use in a cosmetic composition that rupture when the composition is rubbed on to a substrate on the human body thereby releasing the benefit agent contained therein.

### Background of the Invention

There are many benefit agents (such as sunscreens, skin lightening agents, moisturizers, emollients, perfumes, flavours, oils etc.) that are incorporated in cosmetic compositions and/or are delivered to the external surface of the human body through cosmetic compositions. Problems exist with such compositions in that many of these benefit agents may react with other components of the composition thereby limiting their stability in the composition. Such problems have been overcome in the past by incorporating benefit agents through encapsulation techniques. These techniques ensure that the benefit agents do not come in contact with other reactive components of the composition until use. In particular, shear sensitive enscapsulates have been developed. Shear sensitive encapsulates have to be so configured that under normal conditions of manufacture, transportation and storage, the capsules remain intact and do not rupture or break down. However, during use, when the composition is sheared on to the desired substrate (e.g. when the composition is rubbed onto the skin), the capsules rupture and release the benefit agents. Thus the capsules provide the desired stability until the point of use.

EP 0 499 619 (Alkermes, 1992) describes a method for producing protein microspheres comprising the step of contacting a prolamine solution containing at least one type of prolamine with a second liquid which is of limited miscibility with the prolamine solution. This publication is directed to the preparation of biodegradable microcapsules and does not provide for shear-sensitive encapsulates.

US 6,248,268. (XC Corp., 2001) describes microparticles of a thermally gelled polysaccharide prepared by spraying a composition comprising thermally-gelling polysaccharides and an aqueous medium into ambient air to produce aerial gelled microparticles. This publication, is directed to the preparation of beads of specific size and properties for gel chromatographic purposes and does not describe beads suitable for cosmetic purposes. Furthermore the beads are prepared such that they are resistant to shear.

US 6,231,878 (Shiseido, 2001) describes an external treatment composition for treating hair or skin, said external treatment composition comprising a water-containing composition comprising as essential ingredients (i) microcapsules in (ii) a polyhydric alcohol, wherein the microcapsules encapsulate a hydrophobic component, the microcapsules are composed of a gelatin film swollen with water, the microcapsules have partilce sizes of 0.1 to 50 micrometer and the microspheres have a breaking strength of 10 to 300 g/cm². While this invention describes shear sensitive microcapsules that are used in cosmetic compositions, it is desirable to have larger capsules whose distinct presence is visible to the consumer while providing for all of the other desirable properties.

JP 2000-302662 (Noevir, 2003) describes a cosmetic that is prepared by encapsulating or impregnating (A) capsules made of Agar and a polyvalent metal alginate with (B) a cosmetic ingredient (preferably an oily ingredient) and then dispersing the capsules in (C) a water-soluble gel comprising sodium hydroxide and a C₃-₆ dihydric alcohol. The capsules are breakable with slight shear and can be stably dispersed in water-soluble gels. Although the above invention is directed to providing shear-sensitive capsules that are impregnated with benefit agents, it is desirable to provide for improved delivery systems that do no break down under manufacturing conditions but would break when applied on the skin.

WO 2005/020940 (Beiersdorf AG, 2005) relates to a cosmetic and/or dermatological capsule for cosmetic or dermatological substances comprising a solid, semi-solid or shape-retaining envelope essentially consisting of wax, emulsifiers, natural and or synthetic polymers, and/or of the mixtures thereof. The envelope is so constructed that during the friction and/or distribution of a preparation through skin and/or hair it is molten and/or entirely or partially liquified by shear forces and/or dissolved in the filling and/or in the skin sebum lipids such that the envelope is imperceptible from the other contents of the composition. Although this invention is also directed to providing shear-sensitive capsules, it is desirable to provide for further improved delivery systems.

US 5 545 414 (Abbott Laboratories) discloses a nutritional product having a solid matrix containing protein, fat and carbohydrate having disposed therein particles of dietary fibre encapsulated in zein.

US 6 197 319 B1 (Revlon Consumer Products Corporation) discloses an emulsion cosmetic composition for application to skin containing water and at least one surfactant, wherein the composition also contains a water soluble, protein polysaccharide complex ("PPC") having a net positive or negative charge, which is the reaction product of at least one protein and at least one anionic polysaccharide.

It is thus an object of the present invention to provide a benefit agent delivery system in cosmetic products that enhances the stability of the benefit agent incorporated therein.

It is another object of the present invention to provide a benefit agent delivery,system in cosmetic products that provides for release of the benefit agent upon application of shear.

It is another object of the invention to provide cosmetic or cleansing products that enhance the stability of a benefit agent incorporated therein.

It is another object of the invention to provide cosmetic or cleansing products that can provide for shear triggered release of a benefit agent.

### Summary of the invention

Thus according to one aspect of the invention there is provided a benefit agent delivery system for use in cosmetic products comprising:
(i) a polysaccharide-zein complex;
(ii) a benefit agent; and
(iii) a plasticiser,
wherein the delivery system is in the form of shear sensitive substantially spherical globules.

We have found that the presence of the combination of a polysaccharide with zein in the presence of a plasticiser provides the required balance between stability of the delivery system during manufacture and shear-sensitivity during application to the skin. Furthermore, the mechanical properties of the inventive delivery system are such that the delivery system is stable in the cosmetic product but does not abrade the skin on application.

Optionally the delivery system of the invention further comprises a phosphate buffering agent.

It is further particularly preferred that the delivery system is provided in the form of substantially spherical globules that are shear sensitive.

According to another aspect of the invention there is provided a cosmetic composition comprising:
(A) a benefit agent delivery system comprising a polysaccharide-zein complex, benefit agent to be delivered and a plasticiser; and
(B) a cosmetically acceptable vehicle or detergent active.

An especially desirable form of the cosmetically acceptable vehicle is a skin cream base.

According to another aspect of the invention there is provided a process for the preparation of a benefit agent delivery system comprising the steps of:
(i) mixing a benefit agent and a plasticizer in a hydroalcoholic solution of a polysaccharide and zein to provide a mixture with an ionic strength in the range of 0.03 to 0.08; and
(ii) separating the solvents from the mixture to obtain a powder.

Preferably the process comprises the further steps of:
(iii) dispersing the powder in water at a temperature in the range of 40 to 90°C to obtain a dispersion;
(iv) mixing the dispersion with an aqueous solution of a phosphate buffering agent to form the benefit agent delivery system; and
(v) separating the benefit agent delivery system from the solution.

We have found that such a process allows for provision of the delivery system in the form of globules with controlled size and stability. In particular, we have found that the growth kinetics of the globules formed by the process can be controlled by application of the conditions of temperature and ionic strength specified above.

### Detailed Description of the Invention

The invention provides for a benefit agent delivery system and cosmetic products comprising the delivery system. The delivery system comprises a polysaccharide-zein complex, the benefit agent to be delivered and a plasticiser. The benefit agent delivery system of the invention may be used in any cosmetic product that involves shearing the cosmetic product on to a substrate of the human body e.g. skin, hair, nails, oral cavity (especially the teeth) etc. Cosmetic products for topical application are especially preferred. The cosmetic composition is especially preferred for use on the skin or hair. The cosmetic composition may preferably be in the form of a cream, lotion, or gel for topical application on the skin or hair or in the from of a cleansing composition. The cleansing composition may be in the form of a liquid, gel, powder, bar or any other suitable form.

### The benefit agent delivery system

The desirable size range of the benefit agent delivery system is such that 90% by weight of the delivery system is in the size range of 10 to 1000 microns, more preferably 200 to 400 microns. The benefit agents which may be delivered through the benefit agent delivery system of the invention include but are not limited to oils, extracts, powders, flavours, perfumes, moisturizers, emollients, herbal oils, skin lightening agents, sunscreen agents, or mixtures thereof. The benefit agents may be present in the benefit agent delivery system in amounts in the range of 0.001 to 50%, more preferably 0.001 to 20% by weight of the benefit agent delivery system. The benefit agent delivery system of the invention is preferably present in an amount in the range of 0.1 to 20% by weight of the cosmetic composition.

### Polysaccharide

The polysaccharide present in the polysaccharide-zein complex is preferably derived from agar. Agar is known by various synonyms namely agar-agar and Japan isinglass. Botanical names of agar include *Gelidium amansii, Sphaerococcus Euchema, Gelidium cartilagineum* and *Gracilaria confervoides.* Agar is derived from a seaweed gathered on the coasts of, for example, the East Indies, China, Japan and Mexico. Agar has the CAS number [9002-18-0] .

The fraction of agar which has the greatest gelling ability is called agarose. The other fractions are called agaropectin. Agarose is an alternating copolymer of 3-linked β-D-galactopyranose and 4-linked 3,6-anhydro-a-L-galactopyranose units. Agaropectin has essentially the same structure except that varying amounts of the units in the copolymer are replaced by 4,6-O-(1-carboxyethylidene)-D-galactopyranose or by sulfated or methylated sugar residues. The replacement occurs in such a manner that the alternating sequence of 3-linked-β-D-units and 4-linked α-L-units is maintained. Agar contains glose, a carbohydrate which is a powerful gelatinizing agent. It is precipitated from solution by alcohol. It is, however, soluble in hydroalcoholic solvent media.

The polysaccharide-zein complex preferably comprises a water soluble fraction of agar. In addition, it is also desirable to have a fraction of agar which is soluble in hydroalcoholic solvent medium which fraction of agar is preferably predominantly glose. When present, the fraction of agar soluble in water is preferably present in an amount in the range of 15 to 60% on dry weight basis of the polysaccharide-zein complex. When present, the fraction of agar soluble in hydroalcoholic solvent media is preferably present in an amount in the range of 25 to 70% by dry weight basis of the polysaccharide-zein complex. The alcohol for preparing the hydroalcoholic solvent may be ethanol, methanol or isopropyl alcohol. The preferred alcohol for preparing the hydroalcoholic solvent media is isopropyl alcohol. The preferred hydroacoholic solvent media comprises isopropyl alcohol in an amount in the range of 20 to 90% by weight of the hydroalcoholic solvent media.

### Zein

Zein is a water insoluble prolamine from corn gluten. It is a protein which is highly resistant to attack by bacteria. It has been extensively used in the preparation of various food products such as candy or flavours. It is insoluble in water and insoluble in anhydrous alcohol but is soluble in hydroalcoholic solvent media. This property is believed to be due to the presence of the amino acids leucine, proline and alanine. Zein also contains the amino acids glutamic acid and glutamine. Zein is preferably present in an amount in the range of 5 to 30% by weight of the polysaccharide-zein complex.

### Process for preparation of the polysaccharide-zein complex

The polysaccharide-zein complex is preferably prepared from a hydroalcoholic solution of the polysaccharide and the zein (preferably in a mix with the benefit agent and plasticizer). Preferably, carefully measured amounts of an aqueous solution of the polysaccharide agar and a hydroalcoholic solution of glose is used in preparing the complex along with zein. The ionic strength (I_{c}) of the solution is so adjusted such that it is in the range of 0.03 to 0.08. The ionic strength is defined by the equation I_{c} = ¼ Σ_{CB} z_{B}² where C_{B} is the concentration of the ion B present and z_{B} is the charge of the ion B present and the ionic strength is summed over all the ions present. The pH of the mixture is preferably kept in the range of 4 to 6. The polysaccharide-zein complex is then separated from the solvent media preferably by evaporation and drying to remove the solvents (i.e., water and alcohol) to obtain a dry powder.

### Plasticiser

The delivery system of the invention comprises a plasticizer which is preferably an oil, more preferably a vegetable oil. A synthetic plasticizer may also be used e.g. dibutyl phthalate. Suitable oils may be from vegetable or plant sources, preferred oils being castor oil, peanut oil, sesame oil, sunflower oil, safflower oil, or a mixture thereof. The plasticizer is preferably present in an amount in the range of 2 to 10% by weight of the delivery system.

### Phosphate buffering agent

The benefit agent delivery system of the invention optionally comprises a phosphate buffering agent. The phosphate buffering agent preferably produces a pH of between 6 and 8, more preferably about 7, when dissolved in water.

*Process for the preparation of* the benefit agent delivery system The benefit agent delivery system of the invention can be prepared by:
(i) mixing the benefit agent to be delivered, the plasticizer and the polysaccharide-zein complex in water at a temperature in the range of 40 to 90°C to prepare a dispersion;
(ii) mixing the dispersion with an aqueous solution of a phosphate buffering agent, to form the benefit agent delivery system; and
(iii) separating the benefit agent delivery system from the aqueous solution.

It is preferred that the aqueous solution of the phosphate buffering agent has an ionic strength of not less than 1.0. It is also preferred that the temperature of the aqueous solution of the phosphate buffering agent is in the range of 30 to 40°C.

It is also possible to prepare the benefit agent delivery system of the invention starting with the basic raw materials, in which case the process may comprise the steps of:
(i) mixing the benefit agent to be delivered and the plasticizer in a hydro-alcoholic solution of the polysaccharide and the zein such that the ionic strength of the solvent is in the range of 0.03 to 0.08;
(ii) separating the solvents from the mixture to prepare a powder of the polysaccharide-zein complex;
(iii) dispersing the powder in water at a temperature in the range of 40 to 90°C to prepare a dispersion;
(iv) mixing the dispersion with an aqueous solution of a phosphate buffering agent to form the benefit agent delivery system; and
(v) separating the benefit agent delivery system from the aqueous solution.

### Cosmetically acceptable vehicle

The cosmetic composition comprises a cosmetically acceptable vehicle to act as a diluant, dispersant or carrier for the benefit agent delivery system present in the composition, so as to facilitate distribution of the system when the composition is applied to the desired substrate e.g. skin, hair, scalp, or teeth.

The cosmetically acceptable vehicle may suitably comprise one or more of a liquid or solid emollient, solvent, humectant, thickener and powder. Examples of each of these types of material, which can be used singly or as mixtures, are as follows:

Emollients include glycerine, stearyl alcohol, glyceryl monoricinoleate, mink oil, cetyl alcohol, isopropyl isostearate, stearic acid, isobutyl palmitate, isocetyl stearate, oleyl alcohol, isopropyl laurate, hexyl laurate, decyl oleate, octadecan-2-ol, isocetyl alcohol, eicosanyl alcohol, behenyl alcohol, cetyl palmitate, silicone oils (such as dimethylpolysiloxane), di-n-butyl sebacate, isopropyl myristate, isopropyl palmitate, isopropyl stearate, butyl stearate, polyethylene glycol, triethylene glycol, lanolin, cocoa butter, corn oil, cotton seed oil, olive oil, palm kernel oil, rape seed oil, safflower seed oil, evening primrose oil, soybean oil, sunflower seed oil, avocado oil, sesame seed oil, coconut oil, arachis oil, castor oil, acetylated lanolin alcohols, petroleum jelly, mineral oil, butyl myristate, isostearic acid, palmitic acid, isopropyl linoleate, lauryl lactate, myristyl lactate, decyl oleate, myristyl myristate, allantoin, and mixtures thereof. Preferred emollients include glycerine, stearyl alcohol, cetyl alcohol, stearic acid, isocetyl stearate, silicone oils, isopropyl myristate, allantoin and mixtures thereof. The emollients are preferably present in an amount of 5 to 40%, more preferably 10 to 30% by weight of the cosmetic composition.

Solvents include ethyl alcohol, isopropanol, acetone, ethylene glycol monoethyl ether, diethylene glycol monobutyl ether, diethylene glycol monoethyl ether and mixtures thereof.

Powders include chalk, talc, Fullers earth, kaolin, starch, gums, colloidal silica sodium polyacrylate, tetra alkyl and/or trialkyl aryl ammonium smectites, chemically modified magnesium aluminium silicate, organically modified montmorillonite clay, hydrated aluminium silicate, fumed silica, carboxyvinyl polymer, sodium carboxymethyl cellulose, ethylene glycol monostearate and mixtures thereof.

The cosmetically acceptable vehicle is preferably present from 10 to 99.9%, more preferably from 50 to 99% by weight of the cosmetic composition, and can, in the absence of other cosmetic adjuncts, form the balance of the composition. Water is generally present as a part of the cosmetically acceptable vehicle and when present, is preferred at levels of 50 to 90% by weight of the cosmetic composition.

### Detergent active:

The detergent active used in the composition may be a soap or a non-soap surfactant or a mixture thereof, but preferably a soap. The detergent active may be present in amounts in the range of 5 to 80%, preferably from 20 to 75% by weight of the cosmetic composition.

### Soap detergents

The term total fatty matter, usually abbreviated to TFM is used to denote the percentage by weight of fatty acid and triglyceride residues present in soaps without taking into account the accompanying cations.

For a soap having 18 carbon atoms, an accompanying sodium cation will generally amount to about 8% by weight of the soap. Other cations may be employed as desired for example zinc, potassium, magnesium, alkyl ammonium and aluminium.

The term soap denotes salts of carboxylic fatty acids. The soap may be derived from any of the triglycerides conventionally used in soap manufacture - consequently the carboxylate anions in the soap may contain from 8 to 22 carbon atoms.

The soap may be obtained by saponifying a fat and/or a fatty acid. The fats or oils generally used in soap manufacture may be tallow, tallow stearines, palm oil, palm stearines, soya bean oil, fish oil, caster oil, rice bran oil, sunflower oil, coconut oil, babassu oil, palm kernel oil, and others. The fatty acids are preferably derived from oils/fats selected from coconut, rice bran, groundnut, tallow, palm, palm kernel, cotton seed, soybean, castor etc. The fatty acid soaps can also be synthetically prepared (e.g. by the oxidation of petroleum or by the hydrogenation of carbon monoxide by the Fischer-Tropsch process). Resin acids, such as those present in tall oil, may be used. Naphthenic acids are also suitable.

Tallow fatty acids can be derived from various animal sources and generally comprise about 1-8% myristic acid, about 21-32% palmitic acid, about 14-31% stearic acid, about 0-4% palmitoleic acid, about 36-50% oleic acid and about 0-5% linoleic acid. A typical distribution is 2.5% myristic acid, 29% palmitic acid, 23% stearic acid, 2% palmitoleic acid, 41.5% oleic acid, and 3% linoleic acid. Other similar mixtures, such as those from palm oil and those derived from various animal tallow and lard are also included.

Coconut oil refers to fatty acid mixtures having an approximate carbon chain length distribution of 8% C8, 7% C10, 48% C12, 17% C14, 8% C16, 2% C18, 7% oleic and 2% linoleic acids (the first six fatty acids listed being saturated). Other sources having similar carbon chain length distributions, such as palm kernel oil and babassu kernel oil, are included within the term coconut oil.

A particularly preferred fatty acid blend consists of 5 to 30% coconut fatty acids and 70 to 95% fatty acids ex hardened rice bran oil. Fatty acids derived from other suitable oils/fats such as groundnut, soybean, tallow, palm, palm kernel, etc. may also be used in other desired proportions.

### Non-Soap detergents

The composition according to the invention may optionally comprise detergent actives, which are generally chosen from anionic, nonionic, cationic, amphoteric or zwitterionic detergent actives. It is preferred that if non-soap detergents are used in the composition of the invention, the non-soap detergent is chosen from anionic or non-ionic detergent actives.

### Optional ingredients

Skin lightening ingredients can be advantageously included in the composition to provide skin lightening benefits. These may include vitamin B3, vitamin B6, vitamin C, vitamin A or their precursors and mixtures. Especially preferred skin lightening benefit agent is vitamin B3 or a derivative thereof e.g niacinamide. Niacinamide is preferably present in amounts of 0.01 to 5%, more preferably 0.1 to 2% by weight of the cosmetic composition. Another preferred vitamin is vitamin B6, especially when used in combination with vitamin B3. Other skin lightening actives known in the art can also be employed in the invention. Non-limiting examples of skin lightening actives useful herein include aloe extract, ammonium lactate, azelaic acid, kojic acid, lactic acid, linoleic acid, magnesium ascorbyl phosphate, 5-octanoyl salicylic acid, 2,4-resorcinol derivatives, 3,5-resorcinol derivatives, salicylic acid, 3,4,5-trihydroxybenzyl derivatives, and mixtures thereof. Skin lightening agents disclosed in WO 2004/105718 *viz.* extracts of plants from the families of *symplocos* or *rubia* may be optionally included in the composition of the invention. The composition preferably comprises from 0.1% to 10%, more preferably from 0.1% to 5%, by weight of a skin lightening ingredient.

The composition of the invention may include an effective amount of a sunscreen or sun-block agent. Organic and inorganic sunscreens/sun-blocks may be suitably employed in the composition. Suitable organic sunscreen agents include 2-ethylhexyl-p-methoxycinnamate, butylmethoxydibenzoylmethane, 2-hydroxy-4-methoxybenzophenone, octyldimethyl-p-aminobenzoic acid and mixtures thereof. A safe and effective amount of sunscreen may be used in the composition. The composition preferably comprises from about 0.1% to about 10%, more preferably from about 0.1% to about 5%, of a sunscreen agent.

Inorganic sun-blocks include, for example, zinc oxide iron oxide, silica, such as fumed silica, and titanium dioxide. Ultrafine titanium dioxide in either of its two forms, namely water-dispersible titanium dioxide and oil-dispersible titanium dioxide is especially suitable for the invention. Water-dispersible titanium dioxide is ultra-fine titanium dioxide, the particles of which are non-coated or which are coated with a material to impart a hydrophilic surface property to the particles. Examples of such materials include aluminium oxide and aluminium silicate. Oil-dispersible titanium dioxide is ultrafine titanium dioxide, the particles of which exhibit a hydrophobic surface property, and which, for this purpose, can be coated with metal soaps such as aluminium stearate, aluminium laurate or zinc stearate, or with organosilicone compounds.

By "ultrafine titanium dioxide" is meant particles of titanium dioxide having an average particle size of less than 100 nm, preferably 70 nm or less, more preferably from 10 to 40 nm and most preferably from 15 to 25 nm.

Ultrafine titanium dioxide is the preferred inorganic sun-block agent. The total amount of sun block that is preferably incorporated in the composition according to the invention is from 0.1 to 5% by weight of the composition.

The compositions of the present invention can comprise a wide range of other optional components. The CTFA Cosmetic Ingredient Handbook, Second Edition, 1992, which is incorporated by reference herein in its entirety, describes a wide variety of non-limiting cosmetic and pharmaceutical ingredients commonly used in the skin care industry, which are suitable for use in the compositions of the present invention. Examples include: antioxidants, binders, biological additives, buffering agents, colorants, thickeners, polymers, astringents, fragrances, humectants, opacifying agents, conditioners, exfoliating agents, pH adjusters, preservatives, natural extracts, essential oils, skin sensates, skin soothing agents, skin healing agents, and mixtures thereof.

### Examples

The invention will now be illustrated with reference to the following non-limiting examples.

Compositions as shown in Table 1 were prepared as creams by mixing the various ingredients at 45°C using a Silverson^{™} highspeed mixer (Silverson Asia Pacific, Singapore). Samples were stored for at least 4 hours at 20°C prior to testing.

For Comparative Example A the benefit agent *viz*. the herbal oil with distinctive odour, was added as such and mixed with the rest of the ingredients using the Silverson^{™} mixer.

In Example 1 the herbal oil with distinctive odour was delivered through the benefit agent delivery system in the form of globules. The globules were added to the composition at 2% by weight of the composition. The benefit agent delivery system comprised agar-zein complex at 50%, phosphate buffer (0.2 M sodium dihydrogen phosphate + 0.2 M sodium hydroxide; giving a pH of 7.0) at 40%, sesame oil as plasticizer at 5% and the balance being colour, preservatives and other minors. The size distribution (determined by sieve analysis) of the globules by weight of the delivery system was as follows:

| | |
|---|---|
| greater than 400 micron | - less than 5%; |
| 400 to 300 micron | - 18 to 20%; |
| 300 to 250 micron | - 28 to 34%; |
| 250 to 200 micron | - 38 to 45%; |
| 200 to 180 micron | - 8 to 13%; and |
| less than 180 micron | - less than 3%. |

**Table 1**

| Ingredients (% w/w) | Comparative Example A | Example 1 |
|---|---|---|
| Stearic Acid | 18.0 | 18.0 |
| Glycerine | 1.0 | 1.0 |
| Cetyl alcohol | 0.5 | 0.5 |
| Potassium hydroxide | 0.6 | 0.6 |
| Preservatives, (methyl and propyl paraben) | 0.4 | 0.4 |
| Other minors(†) | 2.3 | 2.3 |
| Niacinamide | 0.25 | 0.25 |
| Parsol^{™} MCX | 1.25 | 1.25 |
| Parsol^{™} 1789 | 0.4 | 0.4 |
| Benefit agent, (Herbal oil with distinctive odour (‡)) | 0.02 | 0.02 (*) |
| Water | To 100 | To 100 |

| | | |
|---|---|---|
| (t) colour, preservatives, chelating agent (disodium EDTA), silicone oil, allantoin and TiO₂ as sunscreen. (‡) *Kumkumaditailam* supplied by Arya Vaidya Sala (Kottakkal, India) . (*) Delivered through the benefit agent delivery system. | | |

The samples of the creams were tested by a panel of 200 non-expert consumers. The test was a ten day in-use test conducted using standard monadic product test methodology. While seven desirable attributes (overall skin lightening, skin feel, oiliness, roughness, non-sticky nature, spreadability, and moisturization) were comparable between the two samples, the cream of Example 1 was found to be superior in four attributes, namely: distinctive herbal odour, making the skin soft and smooth, lingering ability of the perfume, and non-irritability. The invention thus provides for enhancing the stability of the benefit agent in a cosmetic composition thereby ensuring better delivery of the benefit to the consumer.

## Claims

1. A benefit agent delivery system for use in cosmetic products comprising:
(i) a polysaccharide-zein complex;
(ii) a benefit agent; and'
(iii) a plasticiser.
wherein the delivery system is in the form of shear sensitive substantially spherical globules.

2. A benefit agent delivery system as claimed in claim 1 further comprising a phosphate buffering agent.

3. A benefit agent delivery system as claimed in any one of the preceding claims wherein the polysaccharide is derived from agar.

4. A benefit agent delivery system as claimed in claim 3 wherein the polysaccharide comprises a fraction of agar soluble in water and a fraction of agar soluble in a hydroalcoholic solution.

5. A benefit agent delivery system as claimed in claim 3 wherein the fraction of agar soluble in water and the fraction of agar soluble in hydroalcoholic solution are present in an amount in the range of 15 to 60% and 25 to 70%, respectively on dry weight basis with respect to the weight of the polysaccharide-zein complex.

6. A benefit agent delivery system as claimed in claim 4 or claim 5 wherein the hydroalcoholic solution comprises isopropyl alcohol.

7. A benefit agent delivery system as claimed claim 6 wherein the hydroalcoholic solution comprises, isopropyl alcohol in an amount in the range of 20 to 90% by weight of the hydroalcoholic solution.

8. A benefit agent delivery system as claimed in any one of the preceding claims wherein zein is present in an amount in the range of 5 to 30% by weight of the zein-polysaccharide complex.

9. A benefit agent delivery system as claimed in any one of the preceding claims wherein the polysaccharide-zein complex is present in amount in the range of 25 to 60% by weight of the delivery system.

10. A benefit agent delivery system as claimed in any one of claims 2 to 9 wherein the phosphate buffering agent is present in an amount in the range of 30 to 60% by weight of the delivery system.

11. A benefit agent delivery system as claimed in any one of claims 2 to 10 wherein the phosphate buffering system dissolves in water to give a pH in the range of 6 to 8.

12. A benefit agent delivery system as claimed in any one of the preceding claims wherein the plasticiser is a vegetable oil.

13. A benefit agent delivery system as claimed in any one of the preceding claims wherein the plasticiser is present in an amount in the range of 2 to 10% by weight of the delivery system.

14. A benefit agent delivery system as claimed in any one of the preceding claims wherein the benefit agent to be delivered is present in an amount in the range of 0.0001 to 20% by weight of the delivery system.

15. A benefit agent delivery system as claimed in any one of the preceding claims wherein the benefit agent is chosen from the group consisting of: oils, extracts, powders, flavours, perfumes, moisturizers, emollients, herbal oils, skin lightening agents, sunscreen agents and mixtures thereof.

16. A benefit agent delivery system as claimed in any one of the preceding claims wherein at least 90% by weight of the benefit agent delivery system is in the form of globules with a size in the range of 200 to 400 microns.

17. A cosmetic or cleansing composition comprising:
(A) benefit agent delivery system according to any one of the preceding claims; and
(B) a cosmetically acceptable vehicle or detergent active.

18. A cosmetic or cleansing composition as claimed in claim 17 wherein the benefit agent delivery system is present in an amount in the range of 0.1 to 20% by weight of the composition.

19. A cosmetic or cleansing composition as claimed in claim 17 or 18 wherein the cosmetically acceptable vehicle is present in an amount in the range of 10 to 99.9% by weight of the composition.

20. A cosmetic or cleansing composition as claimed in any one of claims 17 to 19 wherein the composition further comprises one or more skin lightening agents in an amount in the range of 0.1 to 5% by weight of the composition.

21. A process for the preparation of a benefit agent delivery system for use in cosmetic or cleansing products, the process comprising the steps of:
(i) mixing a benefit agent and a plasticizer in a hydro-alcoholic solution of a polysaccharide and zein to provide a mixture with an ionic strength in the range of 0.03 to 0.08; and
(ii) separating the solvents from the mixture to obtain a powder.

22. A process as claimed in claim 21 further comprising the steps of:
(iii) dispersing the powder in water at a temperature in the range of 40 to 90°C to obtain a dispersion;
(iv) mixing the dispersion with an aqueous solution of a phosphate buffering agent to form the benefit agent delivery system; and
(v) separating the benefit agent delivery system from the solution.

23. A process as claimed in claim 21 or claim 22 wherein the water and the alcohol are separated from the mixture in step (ii) by drying.

24. A process as claimed in claim 22 or claim 23 wherein the ionic strength of the aqueous solution of the phosphate buffering agent in step (iv) is greater than one.

25. A process as claimed in any one of claims 22 to 24 wherein the temperature of the aqueous solution of the phosphate buffering agent in step (iv) is in the range of 30 to 40°C.

## Patentansprüche

1. Abgabesystem für Mittel mit günstigen Eigenschaften zur Verwendung in kosmetischen Produkten, umfassend:
(i) einen Polysaccharid-Zein-Komplex;
(ii) ein Mittel mit günstigen Eigenschaften; und
(iii) einen Weichmacher,
wobei das Abgabesystem in der Form von gegenüber Scherung empfindlichen, im Wesentlichen sphärischen Kügelchen ist.

2. Abgabesystem für Mittel mit günstigen Eigenschaften, wie es in Anspruch 1 beansprucht wird, das außerdem ein Phosphat-Puffermittel umfasst.

3. Abgabesystem für Mittel mit günstigen Eigenschaften, wie es in einem der vorangehenden Ansprüche beansprucht wird, wobei das Polysaccharid von Agar abgeleitet ist.

4. Abgabesystem für Mittel mit günstigen Eigenschaften, wie es in Anspruch 3 beansprucht wird, wobei das Polysaccharid eine Agarfraktion, die in Wasser löslich ist, und eine Agarfraktion, die in wässrig-alkoholischer Lösung löslich ist, umfasst.

5. Abgabesystem für Mittel mit günstigen Eigenschaften, wie es in Anspruch 3 beansprucht wird, wobei die Agarfraktion, die in Wasser löslich ist, und die Agarfraktion, die in wässrig-alkoholischer Lösung löslich ist, in einer Menge im Bereich von 15 bis 60 % bzw. 25 bis 70 % auf Trockengewichtsbasis, bezogen auf das Gewicht des Polysaccharid-Zein-Komplexes, vorliegen.

6. Abgabesystem für Mittel mit günstigen Eigenschaften, wie es in Anspruch 4 oder Anspruch 5 beansprucht wird, wobei die wässrig-alkoholische Lösung Isopropylalkohol umfasst.

7. Abgabesystem für Mittel mit günstigen Eigenschaften, wie es in Anspruch 6 beansprucht wird, wobei die wässrig-alkoholische Lösung Isopropylalkohol in einer Menge im Bereich von 20 bis 90 Gew.-% der wässrig-alkoholischen Lösung umfasst.

8. Abgabesystem für Mittel mit günstigen Eigenschaften, wie es in einem der vorangehenden Ansprüche beansprucht wird, wobei Zein in einer Menge im Bereich von 5 bis 30 Gew.-% des Zein-Polysaccharid-Komplexes vorliegt.

9. Abgabesystem für Mittel mit günstigen Eigenschaften, wie es in einem der vorangehenden Ansprüche beansprucht wird, wobei der Polysaccharid-Zein-Komplex in einer Menge im Bereich von 25 bis 60 Gew.-% des Abgabesystems vorliegt.

10. Abgabesystem für Mittel mit günstigen Eigenschaften, wie es in einem der Ansprüche 2 bis 9 beansprucht wird, wobei das Phosphat-Puffermittel in einer Menge im Bereich von 30 bis 60 Gew.-% des Abgabesystems vorliegt.

11. Abgabesystem für Mittel mit günstigen Eigenschaften, wie es in einem der Ansprüche 2 bis 10 beansprucht wird, wobei das Phosphat-Puffersystem sich in Wasser unter Erzielung eines pH im Bereich von 6 bis 8 löst.

12. Abgabesystem für Mittel mit günstigen Eigenschaften, wie es in einem der vorangehenden Ansprüche beansprucht wird, wobei der Weichmacher ein pflanzliches Öl ist.

13. Abgabesystem für Mittel mit günstigen Eigenschaften, wie es in einem der vorangehenden Ansprüche beansprucht wird, wobei der Weichmacher in einer Menge im Bereich von 2 bis 10 Gew.-% des Abgabesystems vorliegt.

14. Abgabesystem für Mittel mit günstigen Eigenschaften, wie es in einem der vorangehenden Ansprüche beansprucht wird, wobei das abzugebende Mittel mit günstigen Eigenschaften in einer Menge im Bereich von 0,0001 bis 20 Gew.-% des Abgabesystems vorliegt.

15. Abgabesystem für Mittel mit günstigen Eigenschaften, wie es in einem der vorangehenden Ansprüche beansprucht wird, wobei das Mittel mit günstigen Eigenschaften ausgewählt ist aus der Gruppe, bestehend aus Ölen, Extrakten, Pulvern, Aromen, Parfüms, Feuchthaltemitteln, Emollienzien, Kräuterölen, Haut aufhellenden Mitteln, Sonnenschutzmitteln und Gemischen davon.

16. Abgabesystem für Mittel mit günstigen Eigenschaften, wie es in einem der vorangehenden Ansprüche beansprucht wird, wobei wenigstens 90 Gew.-% des Abgabesystems für Mittel mit günstigen Eigenschaften in der Form von Kügelchen mit einer Größe im Bereich von 200 bis 400 Mikrometer sind.

17. Kosmetische oder Reinigungs-Zusammensetzung, umfassend:
(A) Abgabesystem für Mittel mit günstigen Eigenschaften nach einem der vorangehenden Ansprüche, und
(B) ein kosmetisch verträgliches Vehikel oder einen Detergent-Wirkstoff.

18. Kosmetische oder Reinigungs-Zusammensetzung, wie sie in Anspruch 17 beansprucht wird, wobei das Abgabesystem für Mittel mit günstigen Eigenschaften in einer Menge im Bereich von 0,1 bis 20 Gew.-% der Zusammensetzung vorliegt.

19. Kosmetische oder Reinigungs-Zusammensetzung, wie sie in Anspruch 17 oder 18 beansprucht wird, wobei das kosmetisch verträgliche Vehikel in einer Menge im Bereich von 10 bis 99,9 Gew.-% der Zusammensetzung vorliegt.

20. Kosmetische oder Reinigungs-Zusammensetzung, wie sie in einem der Ansprüche 17 bis 19 beansprucht wird, wobei die Zusammensetzung außerdem ein Haut aufhellendes Mittel oder mehrere Haut aufhellende Mittel in einer Menge im Bereich von 0,1 bis 5 Gew.-% der Zusammensetzung umfasst.

21. Verfahren zur Herstellung eines Abgabesystems für Mittel mit günstigen Eigenschaften zur Verwendung in kosmetischen oder Reinigungsprodukten, wobei das Verfahren die folgenden Schritte umfasst:
(i) Vermischen eines Mittels mit günstigen Eigenschaften und eines Weichmachers in einer wässrig-alkoholischen Lösung eines Polysaccharids und Zein unter Bereitstellung eines Gemisches mit einer Ionenstärke im Bereich von 0,03 bis 0,08, und
(ii) Abtrennen der Lösungsmittel aus dem Gemisch unter Erhalt eines Pulvers.

22. Verfahren, wie es in Anspruch 21 beansprucht wird, das außerdem die folgenden Schritte umfasst:
(iii) Dispergieren des Pulvers in Wasser bei einer Temperatur im Bereich von 40 bis 90 °C unter Erhalt einer Dispersion;
(iv) Mischen der Dispersion mit einer wässrigen Lösung eines Phosphat-Puffermittels unter Bildung des Abgabesystems für Mittel mit günstigen Eigenschaften; und
(v) Abtrennen des Abgabesystems für Mittel mit günstigen Eigenschaften von der Lösung.

23. Verfahren, wie es in Anspruch 21 oder Anspruch 22 beansprucht wird, wobei das Wasser und der Alkohol in Schritt (ii) durch Trocknung von dem Gemisch abgetrennt werden.

24. Verfahren, wie es in Anspruch 22 oder Anspruch 23 beansprucht wird, wobei die Ionenstärke der wässrigen Lösung des Phosphat-Puffermittels in Schritt (iv) größer als 1 ist.

25. VerFahren, wie es in einem der Ansprüche 22 bis 24 beansprucht wird, wobei die Temperatur der wässrigen Lösung des Phosphat-Puffermittels in Schritt (iv) im Bereich von 30 bis 40 °C liegt.

## Revendications

1. Système de libération d'un agent bénéfique destiné à être utilisé dans des produits cosmétiques comprenant :
(i) un complexe polysaccharide-zéine ;
(ii) un agent bénéfique ; et
(iii) un plastifiant,
dans lequel le système de libération se présente sous la forme de globules sensiblement sphériques sensibles au cisaillement.

2. Système de libération d'un agent bénéfique selon la revendication 1 comprenant en outre un agent tampon à base de phosphate.

3. Système de libération d'un agent bénéfique selon l'une quelconque des revendications précédentes dans lequel le polysaccharide est dérivé de l'agar-agar.

4. Système de libération d'un agent bénéfique selon la revendication 3 dans lequel le polysaccharide comprend une fraction d'agar-agar soluble dans l'eau et une fraction d'agar-agar soluble dans une solution hydroalcoolique.

5. Système de libération d'un agent bénéfique selon la revendication 3 dans lequel la fraction d'agar-agar soluble dans l'eau et la fraction d'agar-agar soluble dans une solution hydroalcoolique sont présentes en une quantité se situant dans la gamme allant respectivement de 15 à 60 % et de 25 à 70 %, sur la base de leur poids sec par rapport au poids du complexe polysaccharide-zéine.

6. Système de libération d'un agent bénéfique selon la revendication 4 ou la revendication 5
dans lequel la solution hydroalcoolique comprend de l'alcool isopropylique.

7. Système de libération d'un agent bénéfique selon la revendication 6 dans lequel la solution hydroalcoolique comprend de l'alcool isopropylique en une quantité se situant dans la gamme allant de 20 à 90 % en poids de la solution hydroalcoolique.

8. Système de libération d'un agent bénéfique selon l'une quelconque des revendications précédentes dans lequel la zéine est présente en une quantité se situant dans la gamme allant de 5 à 30 % en poids du complexe zéine-polysaccharide.

9. Système de libération d'un agent bénéfique selon l'une quelconque des revendications précédentes dans lequel le complexe polysaccharide-zéine est présent en une quantité se situant dans la gamme allant de 25 à 60 % en poids du système de libération.

10. Système de libération d'un agent bénéfique selon l'une quelconque des revendications 2 à 9
dans lequel l'agent tampon à base de phosphate est présent en une quantité allant de 30 à 60 % en poids du système de libération.

11. Système de libération d'un agent bénéfique selon l'une quelconque des revendications 2 à 10
dans lequel le système tampon à base de phosphate se dissout dans de l'eau pour donner un pH dans la gamme allant de 6 à 8.

12. Système de libération d'un agent bénéfique selon l'une quelconque des revendications précédentes dans lequel le plastifiant est une huile végétale.

13. Système de libération d'un agent bénéfique selon l'une quelconque des revendications précédentes dans lequel le plastifiant est présent en une quantité se situant dans la gamme allant de 2 à 10 % en poids du système de libération.

14. Système de libération d'un agent bénéfique selon l'une quelconque des revendications précédentes dans lequel l'agent bénéfique à libérer est présent en une quantité allant de 0,0001 à 20 % en poids du système de libération.

15. Système de libération d'un agent bénéfique selon l'une quelconque des revendications précédentes dans lequel l'agent bénéfique est choisi dans le groupe constitué par des huiles, des extraits, des poudres, des arômes, des fragrances, des hydratants, des émollients, des huiles végétales, des agents éclaircissants de la peau, des écrans solaires et des mélanges de ceux-ci.

16. Système de libération d'un agent bénéfique selon l'une quelconque des revendications précédentes dans lequel au moins 90 % en poids du système de libération d'un agent bénéfique se présentent sous la forme de globules ayant une taille allant de 200 à 400 micromètres.

17. Composition cosmétique ou nettoyante comprenant :
(A) un système de libération d'un agent bénéfique selon l'une quelconque des revendications précédentes ; et
(B) un véhicule ou un ingrédient actif détergent acceptable sur le plan cosmétique.

18. Composition cosmétique ou nettoyante selon la revendication 17 dans laquelle le système de libération d'un agent bénéfique est présent en une quantité se situant dans la gamme allant de 0,1 à 20 % en poids de la composition.

19. Composition cosmétique ou nettoyante selon la revendication 17 ou 18 dans laquelle le véhicule acceptable sur le plan cosmétique est présent en une quantité se situant dans la gamme allant de 10 à 99,9 % en poids de la composition.

20. Composition cosmétique ou nettoyante selon l'une quelconque des revendications 17 à 19 dans laquelle la composition comprend en outre un ou plusieurs agent(s) éclaircissant(s) de la peau en une quantité se situant dans la gamme allant de 0,1 à 5 % en poids de la composition.

21. Procédé de préparation d'un système de libération d'un agent bénéfique destiné à être utilisé dans des produits cosmétiques ou nettoyants, le procédé comprenant les étapes consistant à :
(i) mélanger un agent bénéfique et un plastifiant dans une solution hydroalcoolique d'un polysaccharide et d'une zéine pour former un mélange ayant une force ionique se situant dans la gamme allant de 0,03 à 0,08, et
(ii) séparer les solvants du mélange pour obtenir une poudre.

22. Procédé selon la revendication 21 comprenant en outre les étapes consistant à :
(iii) disperser la poudre dans de l'eau à une température se situant dans la gamme allant de 40 à 90 °C pour obtenir une dispersion ;
(iv) mélanger la dispersion avec une solution aqueuse d'un agent tampon à base de phosphate pour former un système de libération d'un agent bénéfique ; et
(v) séparer le système de libération d'un agent bénéfique de la solution.

23. Procédé selon la revendication 21 ou la revendication 22 dans lequel l'eau et l'alcool sont séparés du mélange dans l'étape (ii) par séchage.

24. Procédé selon la revendication 22 ou la revendication 23 dans lequel la force ionique de la solution aqueuse de l'agent tampon à base de phosphate dans l'étape (iv) est supérieure à un.

25. Procédé selon l'une quelconque des revendications 22 à 24 dans lequel la température de la solution aqueuse de l'agent tampon à base de phosphate dans l'étape (iv) se situe dans la gamme allant de 30 à 40 °C.
